# EUROPEAN PATENT APPLICATION

(11) **EP 2 156 853 A1**
(43) Date of publication of application: **24.02.2010**
(21) Application number: 08014667.3
(22) Date of filing: 19.08.2008
(51) Int. Cl.: A61M 1/00, A61F 13/20

(54) **Medical product for treatment of sinusitis**

(71) Applicant: Aesculap AG, 78532 Tuttlingen (DE)
(72) Inventor: König, Silke, 78628 Rottweil (DE); Odermatt, Erich, 8200 Schaffhausen (CH)
(74) Representative: Patentanwälte Ruff, Wilhelm, Beier, Dauster & Partner

(57) **Abstract**

The invention relates to a medical product used for treatment of sinusitis and comprisins an absorption body (12) for bodily fluids.

## Description

The invention relates to a medical product for treatment of sinusitis, and a set for producing the medical product.

Sinusitis, or inflammation of the paranasal sinuses, is one of the most common clinical pictures presented in the population. Approximately 15% of the population of the western industrialized nations suffers from chronic inflammation of the paranasal sinuses. Those affected almost always feel very unwell. Typical symptoms include headache, cough, fever, restricted breathing through the nose, and an impaired sense of smell and taste.

Sinusitis often develops from rhinitis, when the discharge of secretions from the paranasal sinuses is obstructed by swelling of the mucous membranes or by anatomical circumstances. The resulting accumulation of secretions represents an ideal breeding ground for microorganisms. Sinusitis is in most cases triggered by viruses, for example rhinoviruses, adenoviruses or RS viruses. An impaired immune defence then often leads to a secondary bacterial infection or what is referred to as a bacterial superinfection. The bacterial pathogens are in most cases Haemophilus influenzae and Streptococcus pneumoniae.

Various therapeutic measures are presently available for the treatment of sinusitis. The main aim of therapy is to reduce the inflammation as far as possible and restore the natural mucosal discharge of the paranasal sinuses. Nose drops that reduce swelling are often used. These act quickly on the nasal obstruction, but only for a short time and for a maximum of 8 hours. Moreover, the use of nose drops often results in what is known as a rebound effect, i.e. a reactive swelling of the mucous membrane after the effect wears off. Frequent use of nose drops also poses the risk of rhinitis medicamentosa or a nose disease induced by nose drops. In addition, frequent use of nose drops and nasal sprays poses a risk of habituation.

In addition, antibiotics are also used for treatment of paranasal sinusitis. A disadvantage is that the healing process may be quite protracted despite the administration of antibiotics. Moreover, purulent inflammatory secretions that are already present are not transported away from the paranasal sinuses by this therapeutic measure. In addition, some difficulties are typically associated with the administration of antibiotics.

In particularly serious cases of sinusitis, surgical measures are available, for example in order to permit discharge of secretion that has been rendered difficult by organic elements. For example, an operation can be carried out to remove the polyps, the nasal concha can be made smaller, bone can be scraped off or the nasal septum straightened.

Gauze bandages are also used in some cases to drain the paranasal sinuses. However, the use of gauze bandages has proven awkward and has also been found to be ineffective in carrying off purulent nasal secretions.

The object of the present invention is therefore to make available a medical product for treatment of sinusitis that avoids disadvantages of the therapeutic measures known from the prior art and that especially permits effective and rapid removal of purulent secretions accumulated in the paranasal sinuses.

According to the invention, this object is achieved by a medical product for treatment of sinusitis, comprising an absorption body, for bodily fluids.

The invention makes available a medical product comprising an absorbent shaped body or an absorption body for treatment of sinusitis. Particularly advantageously, the absorption capacity of the absorption body for bodily fluids, in particular for pathological accumulations of fluid, preferably purulent secretions, is sufficiently great to achieve therapeutically satisfactory drainage of purulent paranasal sinuses. This applies in particular to chronically purulent paranasal sinuses. The absorption body can be adapted particularly advantageously in terms of its size and form to the paranasal sinuses of the individual patient.

Drainage within the meaning of the present invention is intended to be understood as the removal of bodily fluids, in particular pathological accumulations of fluids, especially purulent fluids, from the paranasal sinuses.

In a preferred embodiment, the absorption body is designed at least partially, preferably completely, with open pores. If the absorption body is designed completely with open pores, the bodily fluid to be taken up by it can penetrate rapidly into the absorption body. The absorption body can in principle have a pore size of between 200 and 1200 µm, for example of between 200 and 350 µm. The absorption body preferably has a pore size of between 400 and 950 µm, in particular of between 650 and 950 µm. An absorption body made of polyurethane can, for example, have a pore size of between 400 and 600 µm. By contrast, the pore size of an absorption body made of polyvinyl alcohol can be between 200 and 1000 µm.

The absorption body particularly preferably has an absorption capacity for bodily fluids that corresponds to 10 to 100 times, in particular 20 to 40 times, its dry inherent weight. In the case of sinusitis, the bodily fluids to be taken up are generally purulent nasal secretions. To increase the absorbency of the absorption body, it can contain further additives, in particular superabsorbents, for example crosslinked polyacrylic acid salts or crosslinked polyelectrolytes. The advantage of the embodiments described in this section lies in the rapid uptake of large quantities of purulent secretions, and thus of microorganisms, by the absorption body, and these can then be removed from the paranasal sinuses together with the absorption body. This results in what is generally a favourable healing process.

In another embodiment, the absorption body is designed to be elastic or reversibly compressible. This means that the absorption body can be compressed by the action of a force and largely recovers its original shape after termination of the force. In this way, the absorption body can particularly advantageously be inserted in compressed form into the paranasal sinuses and positioned therein, for example with the aid of a delivery sleeve or trocar. After the delivery and positioning of the absorption body, the latter can then deploy or extend again to its original shape. The absorption body can in this way substantially fill the paranasal sinuses and substantially cover the inside walls thereof.

In a further embodiment, the absorption body is a sponge body or foam body, preferably an open-cell sponge body or an open-cell foam body. A sponge body is particularly preferred. A medical sponge body for treatment of abscesses in other body cavities is known from WO 2004/041346 A1, the disclosed content of which with respect to the features of the described sponge body is to be made, by express reference, part of the content of the present description.

In another embodiment, the absorption body is composed of a polymer, in particular a synthetic polymer. The polymer can be a homopolymer or copolymer, in particular a block copolymer. A copolymer within the meaning of the invention is to be understood as a di-, tri-, tetra-polymer, etc. The polymer can also be resorbable, partially resorbable or non-resorbable. The use of a non-resorbable polymer is preferred. The absorption body is preferably made from a polymer from the group polypropylene, polyethylene, polyvinyl alcohol, polyethylene terephthalate, polyurethane, and copolymers thereof.

In a particularly preferred embodiment, the absorption body is made of polyurethane or a polyurethane derivative, in particular polyurethane ether. The polyurethane can be an aliphatic polyurethane. The polyurethane is preferably a linear aliphatic polyurethane. The polyurethane itself can be composed of macromolecular and/or low-molecular-weight aliphatic diols and aliphatic diisocyanates. Macromolecular diols that can be used are, in particular, polycarbonates, in particular 1,6-hexanediol polycarbonate. Low-molecular-weight diols that can be used are, for example, 2,2,4-trimethyl hexanediol, 2,4,4-trimethyl hexanediol and/or 1,4-butanediol. The aliphatic diisocyanates are preferably cycloaliphatic diisocyanates, in particular 4,4-dicyclohexylmethane diisocyanate or 1,4-cyclohexyl diisocyanate. According to the invention, the polyurethane can be produced from various diols and/or diisocyanates. For further possible polyurethanes, reference is made, for example, to DE 36 43 465 A1, DE 33 18 730 A1 and DE 41 07 284 A1, the disclosed content of which is respectively made part of the content of the present invention. Polyurethane is particularly preferred as material for the absorption body by virtue of its biocompatibility.

As has already been mentioned, the absorption body is usually placed in the paranasal sinuses by means of a suitable delivery instrument, for example a trocar. When the absorption body has taken up the bodily fluids located in the paranasal sinuses, or when the absorption capacity of the absorption body is reached, the absorption body can in principle be removed from the paranasal sinuses using a gripping instrument suitable for this purpose, for example with tweezers, dressing forceps or foreign-body forceps.

In a particularly preferred embodiment, the medical product according to the present invention comprises a drainage tube in addition to the absorption body. In other words, the medical product in this embodiment comprises an absorbent shaped body or absorption body and a drainage tube. Preferably, the absorption body comprises a drainage tube. The drainage tube may be directly connected to the absorption body. The drainage tube serves particularly advantageously for withdrawing or draining off the quantities of fluid absorbed by the absorption body. In this way, the original absorption potential or absorption capacity of the absorption body can be restored. In this embodiment, the absorption body can be used particularly advantageously for suction drainage, the suction preferably being generated outside of the paranasal sinuses. The medical product is preferably used in the context of vacuum therapy. For this purpose, an underpressure is generally generated at a free end of the drainage tube, by means of the free end of the drainage tube being attached to a suitable source that generates an underpressure. This is done in most cases by attaching the drainage tube to a suction pump or vacuum pump. In this way, the bodily fluids that are suctioned can be withdrawn rapidly from the absorption body. The vacuum pump is particularly advantageously a portable electronic vacuum pump. In this way, the patient's mobility is not restricted during treatment. The medical product can remain in the paranasal sinuses of the patient for several hours, if appropriate for several days. For instance, the medical product may be replaced after 8 to 72 hours. In principle, the drainage via the drainage tube can take place at defined intervals. However, it is particularly preferable if the bodily fluids absorbed by the absorption body are withdrawn or drained off continuously. The quantities of fluid withdrawn are usually collected in specially provided collecting containers, for example canisters or vacuum flasks. The collecting containers are generally arranged upstream of the aforementioned suction pump or vacuum pump and communicate with the latter via suitable connecting tubes. In order not to contaminate the suction pump or vacuum pump, a filter can be provided between the collecting containers and the suction pump or vacuum pump.

The absorption body is preferably connected in one piece to a drainage tube. In this embodiment, the proximal end of the drainage tube is connected to the absorption body, whereas the distal end of the drainage tube is free and can, for example, be attached to a suction pump, vacuum pump or the like. According to the invention, provision is made in particular for the absorption body to be formed integrally onto a drainage tube. For example, the absorption body can be adhesively bonded, sewn or welded to a drainage tube or can be foamed onto a drainage tube. Part of the drainage tube generally protrudes into the absorption body. For this purpose, the absorption body preferably has a substantially cylindrically shaped through-channel. The latter generally extends approximately centrally through the absorption body, in the longitudinal direction thereof, and expediently has a diameter adapted to the diameter of the drainage tube. The part of the drainage tube protruding into the absorption body or enclosed by the absorption body preferably has openings. In this way, a uniform underpressure can particularly advantageously be generated on the whole of the absorption body. Moreover, more rapid drainage of the fully soaked absorption body is possible via the openings.

The drainage tube itself is preferably made of a material impermeable to liquid. For example, the drainage tube can be a plastic tube, in particular a polyethylene tube.

According to a further embodiment, the medical product comprises a drainage tube formed by a portion of the absorption body enveloped in a liquid-tight manner. The enveloped portion is preferably tubular. The envelope can be a film, in particular an adhesive film. The envelope can be formed by a hot-melt adhesive, for example. The hot-melt adhesive can be resorbable or non-resorbable. Examples of hot-melt adhesives may be selected from the group consisting of polyglycolides, polylactides, polydioxanones, polycaprolactones, copolymers, polymer blends, and polymer mixtures resulting therefrom.

In a further embodiment, the medical product, preferably the absorption body, comprises an irrigation tube. In a particularly preferred embodiment, the medical product, preferably the absorption body, comprises a drainage tube and an irrigation tube. The irrigation tube can be used to irrigate the absorption body. Examples of irrigation liquid that can be used are a saline solution, a buffer solution or an antiinflammatory solution. In order to remove or kill microorganisms, the absorption body can additionally be irrigated using a disinfecting liquid or using a liquid, in particular a solution, that has an antimicrobial action. Alternatively, the irrigation liquid can itself contain disinfecting or antimicrobial substances. To avoid or combat odours, the irrigation liquid can additionally contain odour-inhibiting additives. The absorption body is generally irrigated after the bodily fluids taken up by it have been removed from the absorption body. For further features and details of the irrigation tube, reference is made to the described embodiments of the drainage tube.

In a further embodiment, the absorption body contains antimicrobial substances, antibiotics, disinfectants, odour-inhibiting and/or antiinflammatory substances. For example, in order to inhibit or combat odours, the absorption body can contain cyclodextrins. Examples of antimicrobial substances that can be used are substances from the group including triclosan, polyhexamethylene biguanide (PHMB), zinc, copper and silver, in particular particles, preferably nanoparticles, thereof. The aforementioned substances or materials can be present homogeneously, i.e. uniformly distributed, in the absorption body. According to the invention, it is also possible for the absorption body to have a surface coating composed of the active substances listed in this paragraph.

The absorption body can in principle be present in different forms. Thus, the absorption body can be adapted in form and size starting from a basic form. It is particularly advantageous for the absorption body to be adapted in terms of its length and form to the dimensions of the paranasal sinuses of the individual patient. The absorption body can in principle be spherical, cuboid, square, tubular or cylindrical. It is preferable for the absorption body to be tubular or cylindrical.

The medical product according to the invention can be used to treat acute sinusitis and also chronic sinusitis. In acute sinusitis, the maxillary sinuses and frontal sinuses of the patients are in most cases also affected, whereas chronic sinusitis additionally involves the ethmoidal sinuses. As has already been mentioned, the absorbency properties of the absorption body mean that the medical product is particularly suitable for taking up large quantities of bodily fluids, preferably purulent nasal secretions, which collect in particular over a long period of time in the paranasal sinuses of patients. For this reason, the medical product is suitable in particular for the treatment of chronic sinusitis. According to the invention, it is also possible for the medical product to be used for prevention of sinusitis.

According to a particular preferred embodiment the medical product according to the present invention is designed as a drainage product or drainage article for the treatment of sinusitis.

The present invention also comprises a set for producing a medical product. The set comprises an absorbent shaped body or absorption body for bodily fluids, in particular for collections of purulent secretions in the paranasal sinuses, and preferably a drainage tube. In addition, the set can comprise a delivery instrument, in particular a delivery sleeve or trocar, for delivering the absorption body to the paranasal sinuses and, if appropriate, for placing the absorption body in the paranasal sinuses. Moreover, the set can also comprise an irrigation tube for irrigating the absorption body and/or an underpressure source or vacuum source, for example a suction pump, for accelerated withdrawal of the bodily fluids taken up by the absorption body. To drain the absorption body, an underpressure source or vacuum source is generally attached to a drainage tube. This is usually done at an underpressure or suction of between 500 and 800 mbar (corresponding approximately to 375 mmHg to 600 mmHg). For further features and details, especially of the medical product, absorption body and drainage tube, reference is made to the preceding description.

The present invention also relates to the use of the medical product for the manufacture or production of a drainage product or drainage article for the treatment of sinusitis, in particular of chronic sinusitis. For further features and details of this reference is likewise made to the preceding description.

The invention further relates to the use of an absorbent shaped body or absorption body, and preferably of a drainage tube, for producing a medical product for treatment of sinusitis. For further features and details of this, in particular of the absorption body and drainage tube, reference is likewise made to the preceding description.

Further features and details of the invention will become evident from the following description of preferred embodiments in the form of descriptions of figures. The figures are here made, by express reference, part of the content of the present description. The individual features can be realized either singly or severally in combination. The figures, including the associated description of the figures, serve merely to illustrate the present invention and are not in any way intended to limit said invention.

In the schematic figures:

Figure 1 shows an embodiment of a medical product, designed as a medical sponge body,

Figure 2 shows an embodiment of a medical product with sponge body and drainage tube,

Figure 3 shows an embodiment of a medical product with sponge body, drainage tube and irrigation tube,

Figure 4 shows an embodiment of a medical product in which the absorption body has a tubular portion enveloped in a liquid-tight manner.

### Description of the figures

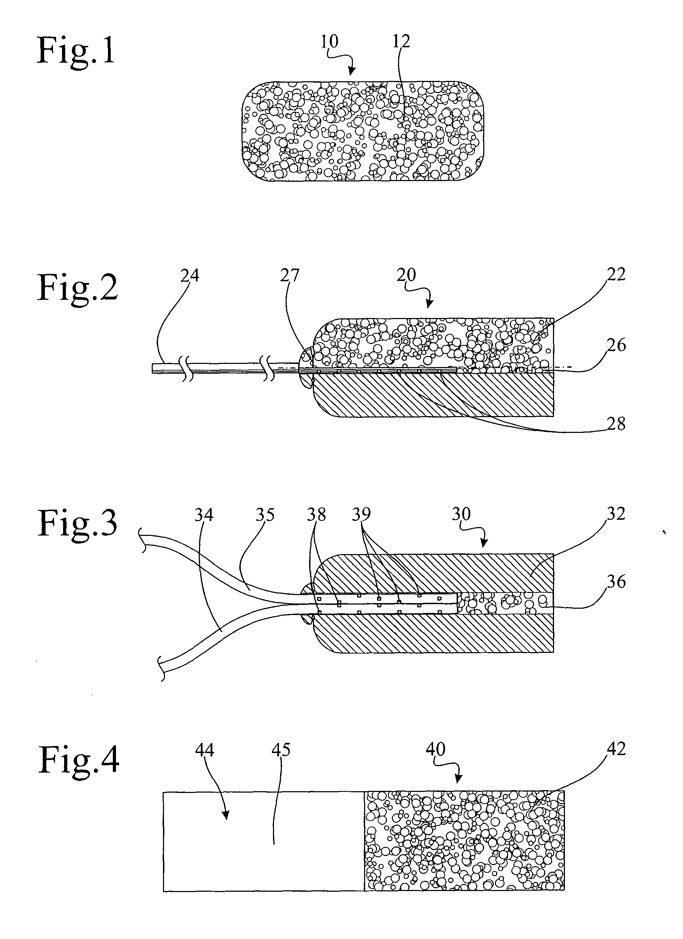

Figure 1 shows a medical product 10 according to the invention, designed as a substantially cylindrical or tubular sponge body 12. The sponge body 12 is made of a polyurethane ether. The sponge body 12 is designed completely with open pores and has a pore size of between 750 and 950 µm. As a result, the sponge body 12 is able to absorb not only nasal secretions, but also solids contained therein, in particular dead mucosal cells and/or microorganisms. As soon as the sponge body 12 is completely filled with nasal secretions, it can be removed from the paranasal sinuses, for example with the aid of a gripping instrument suitable for this purpose.

Figure 2 shows a medical product 20 according to the invention. The product 20 comprises a cylindrical or tubular sponge body 22, made of a polyurethane ether, and a drainage tube 24 made of polyethylene. The sponge body 22 has a cylindrical through-channel 26 which extends approximately centrally in the longitudinal direction of the sponge body 22 and into which the drainage tube 24 partially extends. To secure the sponge body 22 on the drainage tube 24, the sponge body 22 is tied off with a thread 27 at its end directed toward the drainage tube 24. The nasal secretions taken up by the sponge body 22 can be carried off with the aid of the drainage tube 24. This is usually done by applying an underpressure at the free or distal end of the drainage tube 24. For this purpose, the drainage tube 24 is generally attached to a suction pump. The drainage tube 24 also has openings 28 in its wall, as a result of which an underpressure that is as uniform as possible can be generated on the sponge body 22 and, in addition, rapid drainage of the sponge body 22 and thus also of the paranasal sinuses is possible. Within a relatively short time, it is thus possible to remove from the paranasal sinuses large quantities of accumulated nasal secretions and thus also of infectious foci and microbial colonies that weaken the patient's immune system.

The medical product 30 depicted in Figure 3 has a similar structure to the product 20 depicted in Figure 2. The product 30 also has an absorption body 32 designed as a sponge. In addition to a drainage tube 34 made of polyethylene, the product 30 has an irrigation tube 35, likewise made of polyethylene. The tubes 34 and 35 protrude together into a cylindrical through-channel 36 of the sponge body 32. The tubes 34 and 35 have openings 38 and 39, respectively. The through-channel 36 extends approximately centrally through the sponge body 32, in the longitudinal direction thereof. After the sponge body 32 has been drained via the drainage tube 34, the sponge body 32 can be irrigated with the aid of the irrigation tube 35, for example with a disinfecting solution. The irrigated product 30 is in principle suitable for renewed use. For further features, reference is made to the description of Figure 2.

Figure 4 shows a tubular medical product 40 designed as a sponge. Nasal secretions are in this case taken up via the open-pore portion 42 of the product 40. A portion 44 of the product 40 is enveloped in a liquid-tight manner and acts as drainage tube. The envelope 45 is designed as a hot-melt adhesive film based on polyglycolide. For further features, reference is made to the descriptions of the preceding figures.

## Claims

1. Medical product for treatment of sinusitis, comprising an absorption body for bodily fluids.

2. Medical product according to claim 1, **characterized in that** the absorption body is designed at least with open pores.

3. Medical product according to claim 1 or 2, **characterized in that** the absorption body has a pore size of between 200 and 1200 µm.

4. Medical product according to one of the preceding claims, **characterized in that** the absorption body has an absorption capacity for bodily fluids that corresponds to 10 to 100 times its dry inherent weight.

5. Medical product according to one of the preceding claims, **characterized in that** the absorption body is designed to be elastic or reversibly compressible.

6. Medical product according to one of the preceding claims, **characterized in that** the absorption body is a sponge body or foam body.

7. Medical product according to one of the preceding claims, **characterized in that** the absorption body is composed of a polymer from the group polypropylene, polyethylene, polyvinyl alcohol, polyethylene terephthalate, polyurethane, caboxymethylcellulose and copolymers thereof.

8. Medical product according to one of the preceding claims, **characterized in that** the absorption body is made of polyurethane or a polyurethane derivative.

9. Medical product according to one of the preceding claims, **characterized in that** the product further comprises a drainage tube.

10. Medical product according to one of the preceding claims, **characterized in that** the product further comprises an irrigation tube.

11. Medical product according to one of the preceding claims, **characterized in that** the sinusitis is chronic sinusitis.

12. Medical product according to one of the preceding claims, **characterized in that** the product is designed as a drainage product or drainage article for treatment of sinusitis.

13. Set for producing a medical product according to one of the preceding claims, comprising an absorption body for bodily fluids and preferably a drainage tube.

14. Use of a medical product according to the claims 1 to 12 for the manufacture or production of a drainage product or drainage article for treatment of sinusitis.

15. Use of an absorption body, and preferably of a drainage tube, for producing a medical product according to one of the claims 1 to 12.
